# EUROPEAN PATENT SPECIFICATION

(11) **EP 1 531 159 B1**
(45) Date of publication and mention of the grant of the patent: **12.10.2005**
(21) Application number: 03025866.9
(22) Date of filing: 11.11.2003
(51) Int. Cl.: C07K 7/08, A61K 38/04, A61K 38/08, A61P 35/00

(54) **Peptide useful for diagnosis and therapy of tumors**
Peptid für die Diagnose und Therapie von Tumoren
Peptide pour diagnose et thérapie de tumeurs

(43) Date of publication of application: 18.05.2005
(73) Proprietor: Deutsches Krebsforschungszentrum Stiftung des öffentlichen Rechts, 69120 Heidelberg (DE); Ruprecht-Karls-Universität Heidelberg, 69120 Heidelberg (DE)
(72) Inventor: Zitzmann, Sabine, 69214 Eppelheim (DE); Peschke Sigrid, 69493 Hirschberg (DE); Mahmut Miriam, 69469 Weinheim (DE); Mier Walter, 69115 Heidelberg (DE); Haberkorn Uwe, 68723 Schwetzingen (DE)
(74) Representative: Schüssler, Andrea

(56) References cited:
- DATABASE EMBL [Online] 1 June 2003 (2003-06-01) "Conserved hypothetical protein." retrieved from EBI Database accession no. Q8A0B3 XP002271478
- DATABASE EMBL [Online] 6 November 2003 (2003-11-06) "Kif2c-prov protein." retrieved from EBI Database accession no. AAH44976 XP002271479
- DATABASE EMBL [Online] 2 October 2003 (2003-10-02) "Gp48." retrieved from EBI Database accession no. AAQ54982 XP002271480
- DATABASE EMBL [Online] 1 November 1997 (1997-11-01) "Kinesin central motor 1 (XKCM1)" retrieved from EBI Database accession no. Q91636 XP002271481
- DATABASE EMBL [Online] 1 November 1997 (1997-11-01) "Hypothetical ATP-binding protein MG140 homolog ((E07_orf1113)" retrieved from EBI Database accession no. P75033 XP002271482

## Description

The present invention relates to the peptide motif FRPNRAQDYNTN and related motifs characterizing peptides which are capable of specifically binding to tumors, e.g., a prostate tumor. The present invention also relates to various diagnostic and therapeutic uses of peptides containing said motif.

Unfortunately, when imaging or treating tumors, the compounds used (e.g., tracers, chemotherapeutic agents or radionuclides) are unspecifically distributed in the whole organism merely due to their chemical characteristics. However, e.g., for diagnosis of a tumor and potential metastasis specific imaging only of the tumor/metastases is desirable and a prerequisite for selective medical intervention. Thus, the coupling of a tumor specific marker to a tracer is required, otherwise tumor imaging cannot be achieved. In case of tumor therapy, the unspecific distribution of the therapeutic agent, e.g., chemotherapeutic drug, in the organism requires the application of high amounts of the drug in order to achieve a sufficiently high concentration within the organ to be treated resulting in severe side effects and damages of healthy tissue. Nevertheless, often the concentration of the therapeutic drug within the desired organ is quite low, thus, not all tumor cells are killed and an effective treatment is prevented. In order to overcome this problem, for tumor imaging several alternative approaches were tried, e.g., use of tumor specific antibodies (e.g., directed against PSA or CEA), use of unspecific tracers like MIBI or use of tracers allowing to record tumor metabolism (e.g., fluorodeoxyglucose) or use of peptides specific for the expression of tumor receptors (e.g., Octreotid for recording somatostatin receptors). Unfortunately, PET using fluorodeoxyglucose gives with some kinds of tumors (e.g., prostate carcinoma) only a very low accumulation and, thus its application for such kind of tumors is limited. Unfortunately, the approaches for tumor imaging discussed above exhibit a variety of additional disadvantages, e.g., undesired immune responses of the patients when using humanized antibodies, slow accumulation of the antibodies due to their large size etc. Octreotid is only useful for tumors showing expression of SSTR (e.g., neuroendocrine tumors, meningiomas, small cell lung carcinomas).

Therefore, it is the object of the present invention to provide a means for the efficient, reliable and specific imaging of tumors and metastasis as well as therapy which overcomes the disadvantages of the diagnostic and therapeutic approaches presently used.

According to the present invention this is achieved by the subject matters defined in the claims.

A powerful technique utilizing phage display peptide libraries has been used that allows for the selection of peptide sequences with desired binding specifities. In this phage system, 10⁹ different peptides motifs are expressed on the phage surface as peptides fused to phage surface proteins. The desired peptide is selected on its binding to the target cell. By using this approach, a novel peptide, Du-Sigi1 (aa sequence FRPNRAQDYNTN) could be isolated showing specific binding to tumor cells, i.e., it accumulates in the body at/in tumors. Therefore, it is generally possible to visualize tumors and metastasis through use of the peptide of the present invention when coupled to a compound that is detectable upon imaging. Moreover, the peptide of the invention when coupled to a compound exerting a therapeutic effect, e.g., a cytotoxin, can be used for tumor therapy. The peptide of the invention also allows to evaluate the dynamics of tumor progression in a patient and to monitor the effect of therapy.

Therapy/imaging of tumors or metastasis by use of the peptide of the invention exhibits several advantages:
(a) Large amounts of peptides can be easily prepared;
(b) they do not elicit undesired immune responses;
(c) they can be used in combination with already approved drugs and compounds;
(d) the peptide targets the tumor tissue specifically without affecting normal tissue. Thus, in therapy side effects of therapeutic compounds coupled to the peptide are minimized. Accordingly, high concentrations of the therapeutic compound at the site of the tumor can be applied (resulting in efficient killing of the tumor) and at the same time the poisoning of the normal tissue can be reduced; and
(e) as regards the diagnostic aspect, the location and size of the tumor/metastasis can be exactly determined, thus, allowing a more precise planning and carrying out of therapy.

Accordingly, the present invention relates to a peptide with selective binding to tumor cells, wherein said peptide comprises a peptide having the amino acid sequence FRPNRAQDYNTN or an amino acid sequence which differs from the amino acid sequence FRPNRAQDYNTN by one or more conservative amino acid substitutions.

The term "selective binding to tumor cells" as used herein means that, on the one hand, the peptide binds to tumor cells with a stability that is sufficient for therapeutic or diagnostic purposes and, on the other hand, does not bind to non-tumor cells or binds to the non-tumor cells to a substantially lesser extent compared to tumor cells.

The term "conservative amino acid substitutions" involves replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Preferably, the peptide of the invention is characterized by not more than 8 conservative aa substitutions, more preferably by not more than 6 conservative aa substitutions and, even more preferably, by not more than 4 conservative aa substitutions.

The present invention also relates to a peptide with selective binding to tumor cells, wherein said peptide comprises
(a) a peptide having an amino acid sequence which shows at least 60%, preferably at least 80%, more preferably 85% and even more preferably 90% identity to the amino acid sequence FRPNRAQDYNTN without losing its capability of selective binding to a tumor.

The prior art discloses peptides according to the invention but without medical utility (cf. Database EMBL).

For the generation of peptides showing a particular degree of identity to the peptide DU-Sigi1 ,e.g.,genetic engineering can be used to introduce amino acid changes at specific positions of a cloned DNA sequence to identify regions critical for peptide function. For example, site directed mutagenesis or alanine-scanning mutagenesis (introduction of single alanine mutations at every residue in the molecule) can be used. (Cunningham and Wells, Science 244 (1989), 1081-1085) The resulting mutant molecules can then be tested for biological activity using the assay of Example 2.

In the fragment of the peptide of the invention at least 3 contiguous aa, preferably at least 5 contiguous aa, more preferably at least contiguous 7 aa and even more preferably at least 9 contiguous aa of the amino acid sequence FRPNRAQDYNTN are left. The fragment is still capable of selective binding to a tumor cell. For evaluating whether a particular fragment (or derivative of the peptide DU-Sigi1 characterized by substitutions of conservative amino acids) is still capable of selective binding of tumor cells the assay of Example 2 can be used.

A further embodiment of the invention relates to peptides described above which are operatively attached to a therapeutic agent capable of exerting a therapeutic effect on a tumor. Examples of therapeutic agents useful for the present invention include an anticellular agent, chemotherapeutic agent, a radioisotope, a chelator for the binding of certain radioisotopes or a cytotoxin. Preferred cytotoxins are an A chain toxin, a ribosome inactivating protein, α-sarcin, aspergillin, restrictotin, diphtheria toxin, Pseudomonas exotoxin, a bacterial endotoxin or the lipid A moiety of a bacterial endotoxin. Further suitable proteins for tumor therapy are endostatin (for inhibition of tumor growth) or recombinant chimeric toxin PE37/transforming growth factor alpha (TGF-alpha) (for cytotoxicity to tumor cells).

The present invention also relates to polynucleotides encoding a peptide of the invention, preferably fused to secretory leader sequences, as well as expression vectors which are capable of expressing the peptide of the invention and which can be used for gene therapy. Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene theapy are described in the literature and are known to the persons skilled in the art; see, e.g., WO 94/29469 or WO 97/00957.

In a further embodiment, the present invention relates to a pharmaceutical composition containing a peptide, polynucleotide or expression vector of the invention. For administration the compounds of the pharmaceutical composition are preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature and location of the tumor and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the tumor, general health and other drugs being administered concurrently.

An alternative embodiment of the present invention relates to an above disclosed peptide which is linked to a diagnostic agent such as a chelator that is detectable upon imaging. Thus, such a peptide is useful fur tumor diagnosis by imaging, e.g., magnetic resonance imaging (MRI), PET etc.

In a preferred embodiment of the present invention, said diagnostic agent is a paramagnetic ion, radioactive ion or a fluorogenic ion. Specifically, the diagnostic agents utilized in embodiments of the invention include: chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III), erbium (III), iodine¹²³, technetium^{99m}, indium¹¹¹, rhenium¹⁸⁸, rhenium¹⁸⁶, copper⁶⁷, iodine¹³¹, yttrium⁹⁰, iodine¹²⁵, astatine²¹¹, ¹⁷⁷Lutetium and gallium⁶⁷.

The present invention also relates (a) to the use of a peptide, polynucleotide or expression vector of the invention for the preparation of a medicament for the treatment of a tumor and (b) to the use of a peptide of the present invention for the preparation of a diagnostic composition for tumor imaging.

Preferred tumors that can be treated/diagnosed by the compounds of the present invention are prostate tumors, thyroid carcinomas, breast cancer and neuroblastoma.

The present invention is further explained by the following figures which show:
Fig. 1: Binding of ¹²⁵I-DU-Sigi1 to human tumor cell lines and primary endothelial cells
Fig. 2: Binding of DU-Sigi1 to DU-145 prostate tumor cells in vitro
Fig. 3: Competition assay of ¹²⁵I-DU-Sigi1 with unlabeled DU-Sigi1 on DU-145 prostate tumor cells in vitro
Fig. 4: Confocal microscopy with FITC-labeled DU-Sig1 on DU-145 prostate tumor cells in vitro
Fig. 5: Distribution of intravenously injected peptide DU-Sig1 in various organs

The present invention is explained by the following examples.

### Example 1: Selection of a peptide specifically binding to tumor cells

For the selection of tumor specific peptides, 6 selection rounds, so called biopannings, were perfomed on DU-145 prostate carcinoma cells. The phage display library used was the Ph.D.12 phage library from New England Biolabs. Each biopanning was conducted as follows:
For the first round 5 µl of the original library (approx. 5x10¹⁰TU/ml, ~10⁹ different peptide motifs) were added to a cell culture dish with 293-cells (embryonal kidney cells) for a negative selection. After 1 h the medium was collected from the 293 cells, centrifuged for 5 min at 1500 rpm and the supernatant was transfered into a dish with DU-145 cells grown to 90% confluency. After 1 h the medium was removed from the DU-145 cells and the cells were washed 4 x with 10 ml of HBSS(+) + 1 % BSA and 4x with 10 ml of HBSS(-) + 1 % BSA. The cells were then detached with 4 ml of Versene for 5 min and resuspended in 16 ml RPMI 1640 medium and centrifuged 5 min at 1500 rpm. The cell pellet was resuspended in 1 ml HBSS(-) + 1 % BSA into an Eppendorf tube, centrifuged 5 min at 1500 rpm and the supernatend was removed. This wash step was repeated 3 times. Then the cell pellet was lysed with 1 % Triton. 10 µl of the lysate was used for titering. The remaining lysate was added to 50 ml of a log culture of ER2537 bacterial cells and grown 5 h for amplification. The amplified phages were precipitated with PEG (see manufacturer's protocol) and the titer of the amplified phage suspension was determined. For the next biopanning 10¹¹ TU from the previous biopanning were used. 6 biopanning were performed, then single phages clones were amplified and single stranded DNA was isolated for sequencing. From the 24 phages sequenced all phages carried the same peptide: FRPNRAQDYNTN.

### Example 2: Binding of the peptide DU-Sigi1 on various cell types

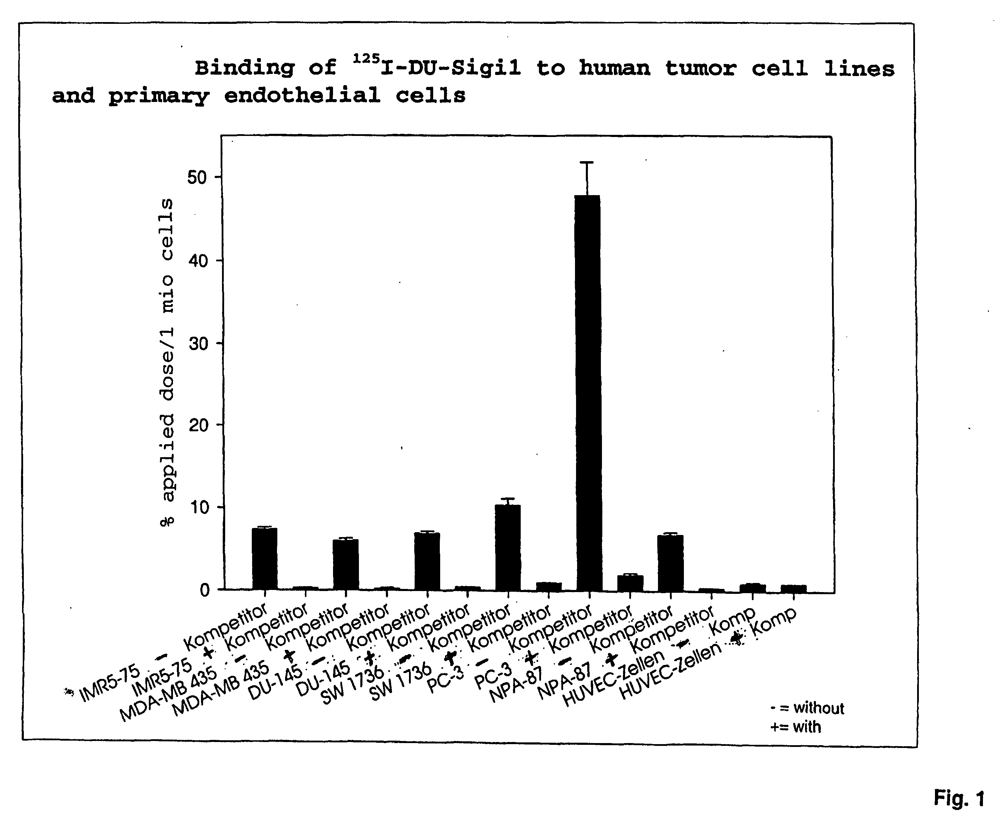

The peptide DU-Sigi1 (FRPNRAQDYNTN) was prepared by chemical synthesis and labelled with ¹²⁵I. For in vitro studies, 200,000 cells (DU-145, human prostate tumor cell line; PC-3, human prostate cell line; HUVEC, human primary endothelial cells, IMR5-75, human neuroblastoma cell line, MDA-MB-435, human breast cancer cell line, SW-1736, human thyroid cancer cell line, NPA-87, human thyroid cancer cell line) were seeded into 6-well plates and cultivated for 24 hours. Then, the medium was replaced by fresh medium (without FCS) and for the plates with competitor, unlabelled peptide was added (10⁻⁴ M) and preincubated for 30 min. Then, ¹²⁵I-labelled peptide was added to the cell culture (1-2 mio cpm/well) and incubated for 10 min. The cells were collected and washed for three times with 1 ml PBS per washing. Then, the cells were lysed with 0.3 M NaOH. Radioactivity of the lysed cells was determined with a gamma counter and radioactivity as percent applied dose per 1 million cells was calculated. It could be shown that the labelled peptide specifically binds to the various tumor cells, but not the primary endothelial cells. Binding can be competitively inhibited (up to 95%). The results are presented in Figure 1.

### Example 3: Binding kinetics of DU-Sigi1 to DU-145 prostate tumor cells in vitro

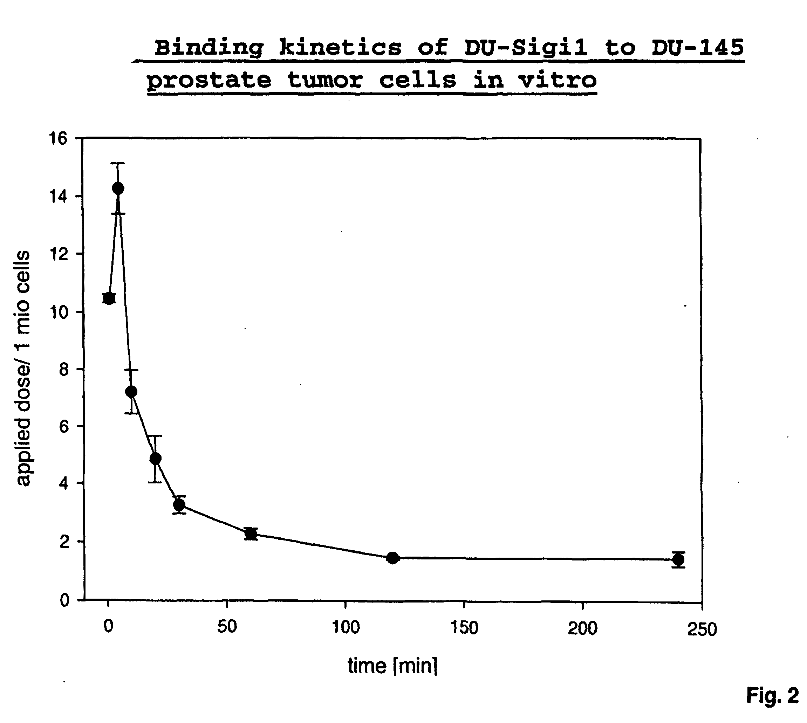

The peptide DU-Sigi1 (FRPNRAQDYNTN) was prepared by chemical synthesis and labelled with ¹²⁵I. 200,000 DU-145 cells (human prostate tumor cell line) were seeded into 6-well plates and cultivated for 24 hours. Then, the medium was replaced by fresh medium (without FCS). ¹²⁵I-labelled peptide was added to the cell culture (1-2 mio cpm/well) and incubated for times from 1 min to 4 h. After the incubation time the cells were collected and washed for three times with 1 ml PBS per washing. Then, the cells were lysed with 0.3 M NaOH. Radioactivity of the lysed cells was determined with a gamma counter and radioactivity as percent applied dose per 1 million cells was calculated. It could be shown that the binding of DU-Sigi1 is rapid and the highest binding rate occurs after 5 min. Then the amount of bound peptide decreases and reaches a basal level of about 1.5 % applied dose/1 mio cells. The same results were obtained with PC-3 cells (prostate tumor cell line, data not shown). The results are presented in Figure 2.

### Example 4: Competition assay of ¹²⁵I-DU-Sigi1 with unlabeled DU-Sigi1 on DU-145 prostate tumor cells in vitro

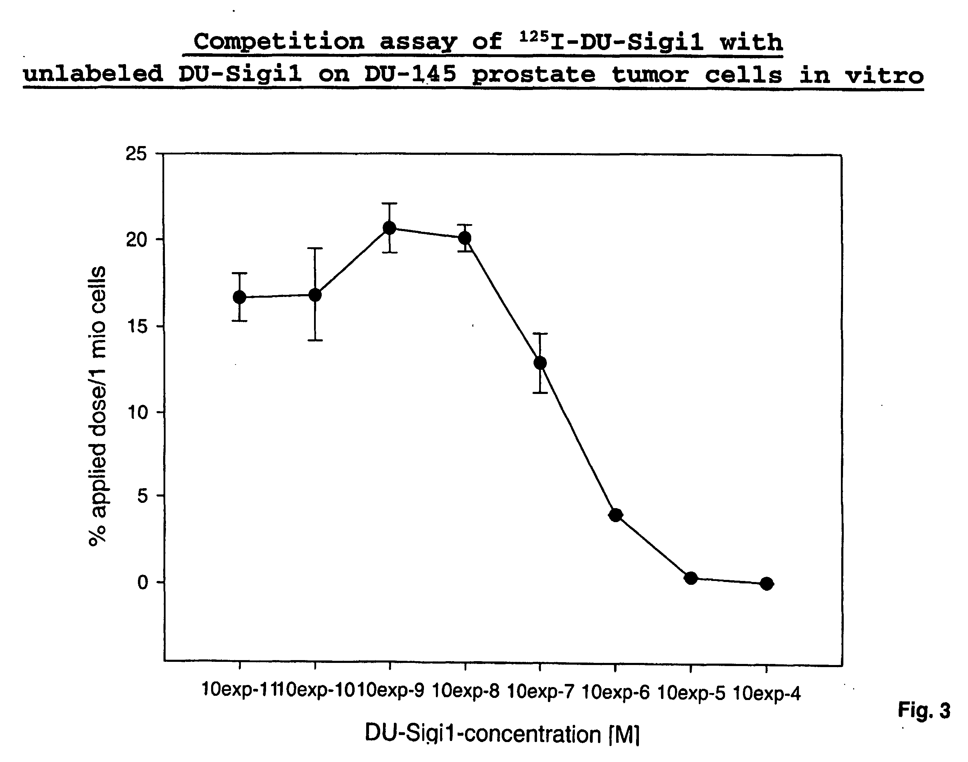

The peptide DU-Sigi1 (FRPNRAQDYNTN) was prepared by chemical synthesis and labeled with ¹²⁵I. 200,000 DU-145 cells (human prostate tumor cell line) were seeded into 6-well plates and cultivated for 24 hours. Then, the medium was replaced by fresh medium (without FCS). Different competitor concentrations (unlabeled DU-Sigi1) were added to the medium 30 min before the ¹²⁵I-labeled peptide was added to the wells (1-2 mio cpm/well) and incubated for 10 min at 37°C.-62, After the incubation time the cells were collected and washed for three times with 1 ml PBS per washing. Then, the cells were lysed with 0.3 M NaOH. Radioactivity of the lysed cells was determined with a gamma counter and radioactivity as percent applied dose per 1 million cells was calculated. It could be shown that concentrations of 10⁻⁴ and 10⁻⁵ inhibit binding of ¹²⁵I-DU-Sigi1 to more than 95%, demonstrating the specificity of the binding of DU-Sigi1. The ID₅₀ is approx. 50 µM. The same results were obtained with PC-3 cells (prostate tumor cell line, data not shown). The results are presented in Figure 3. No competition could be seen with unlabeled octreotide as competitor using the same concentration (data not shown).

### Example 5: Confocal microscopy with FITC-labeled DU-Sigi1 on DU-145 prostate tumor cells in vitro

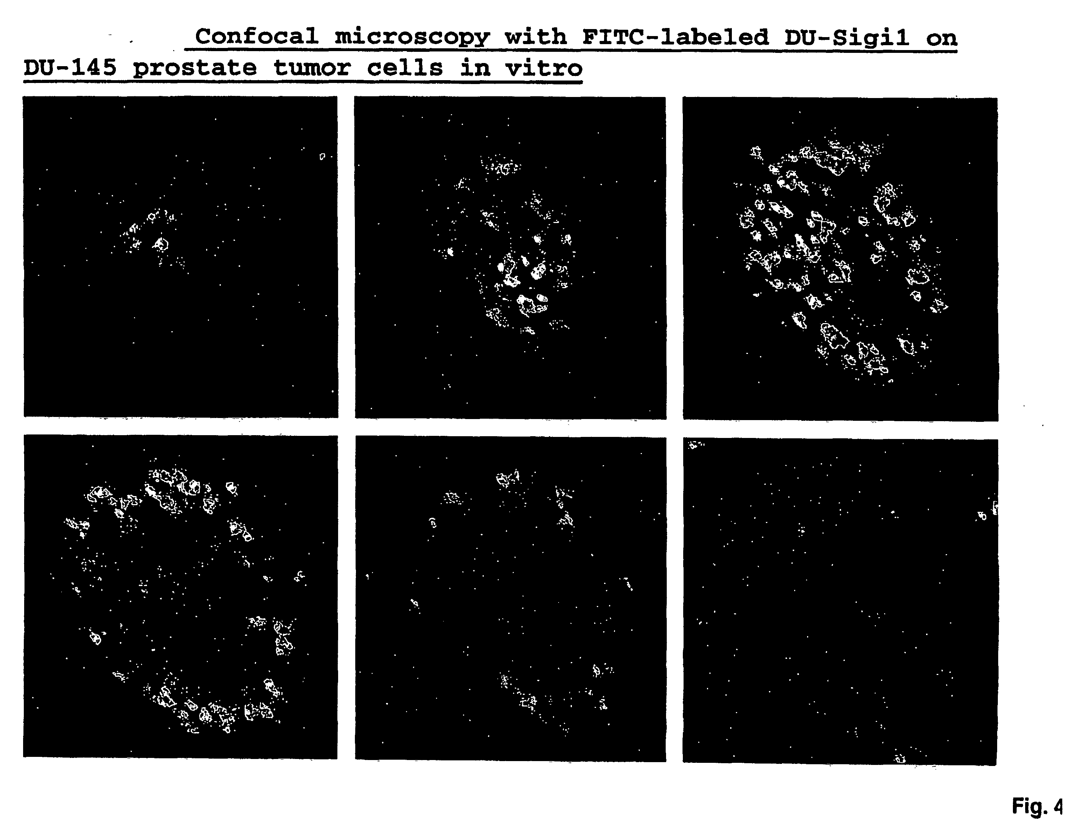

The peptide DU-Sigi1 (FRPNRAQDYNTN) was prepared by chemical synthesis and labeled with FITC. DU-145 cells (human prostate tumor cell line) were seeded subconfluent onto cover slips and cultivated for 24 hours. Then, the medium was replaced by fresh medium (without FCS) and _FITC-DU-Sigi1 was added to the media (10⁻⁵M) and incubated for 10 min at 37°C. After the cover slips were washed with 1 ml medium and the cells were fixed with 2 % formaldehyde for 15 min. After fixation the cells were incubated with TO-PRO-3 (Molecular Probes, Eugene, USA, 1:1000 dilution) for 20 min for cell nucleus staining. Then, the cover slips were placed up side down with a drop of fluorescent mounting medium on a slide and air-dried in the dark for several hours before examining them with a confocal microscope. The results are presented in Fig. 4. It shows a series of images obtained from different layers of a DU-145 cell with Fig. 4A focusing on the very top of the cell and Fig. 4f on the bottom of the cell on the slide. It can be seen that FITC-DU-Sigi1 binds specifically to the membrane of the cells.

### Example 6: Distribution of intravenously injected peptide DU-Sigi1 in various organs

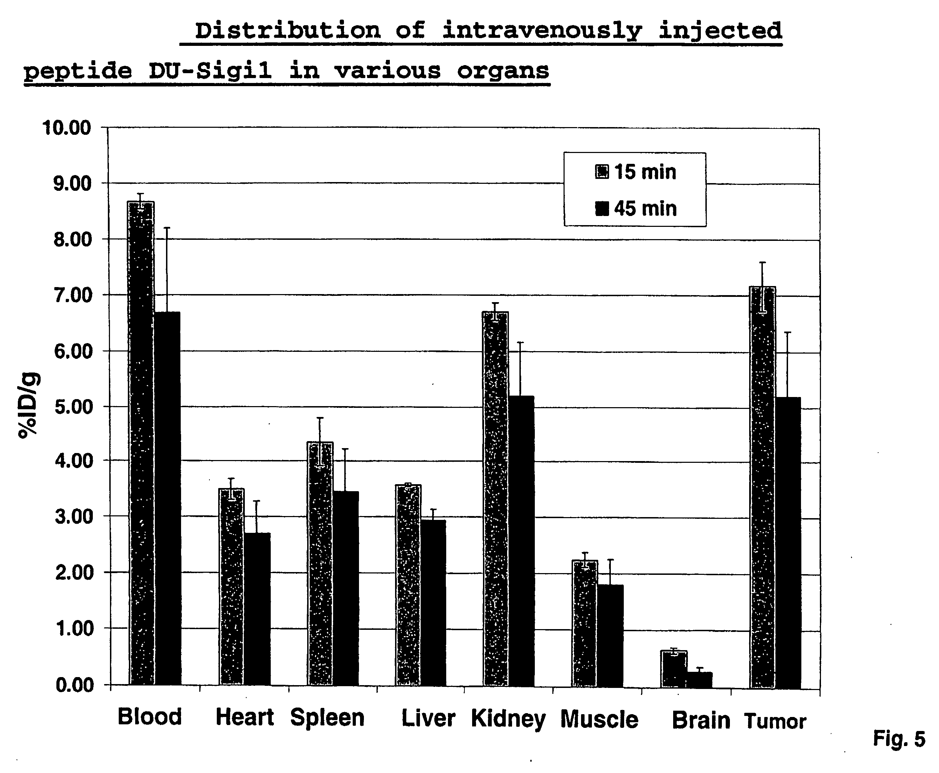

The peptide DU-Sigi1 (FRPNRAQDYNTN) was prepared by chemical synthesis and labeled with ¹³¹I. ¹³¹I labeled peptide DU-Sigi1 (FRPNRAQDYNTN) (28 mio cpm/mouse) was intravenously injected into male nu/nu mice, which subcutaneously carried a human prostate tumor (DU-145). 15 min. and 45 min, respectively, postinjection the mice were sacrificed. The organs were removed, weighed and the radioactivity was determined with a gamma counter. The results are presented in Fig. 5. They show that DU-Sigi1 accumulates in the tumor to a higher extent than in the other organs, with the exeption of the kidney indicating an excretion of the peptide through the kidney. The high blood volumn might be due to the degradation of the peptide by serum peptidases and proteases.

## Claims

1. A peptide for use in tumor therapy being capable of selectively binding to tumor cells, wherein said peptide comprises
(a) a peptide having the amino acid sequence FRPNRAQDYNTN or an amino acid sequence which differs from the amino acid sequence FRPNRAQDYNTN by one or more conservative amino acid substitutions; or
(b) a peptide having an amino acid sequence which shows at least 60% identity to the amino acid sequence of the peptide of (a),
wherein said conservative amino acid substitutions are replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln; replacement of the basic residues Lys, Arg, and His; replacement of the aromatic residues Phe, Tyr, and Trp, and replacement of the small-sized amino acids Ala, Ser, Thr, Met and Gly.

2. The peptide of claim 1 which is operatively attached to a therapeutic agent capable of exerting a therapeutic effect on a tumor.

3. The peptide of claim 2, wherein said therapeutic agent is an anticellular agent, chemotherapeutic agent, a radioisotope or a cytotoxin.

4. The peptide of claim 3, wherein said cytotoxin is an A chain toxin, a ribosome inactivating protein, α-sarcin, aspergillin, restrictotin, diphtheria toxin, Pseudomonas exotoxin, a bacterial endotoxin or the lipid A moiety of a bacterial endotoxin.

5. A polynucleotide encoding the peptide of claim 1.

6. An expression vector containing the polynucleotide of claim 5.

7. A pharmaceutical composition containing a peptide of any one of claims 1 to 4, a polynucleotide of claim 5 or an expression vector of claim 6.

8. The peptide of claim 1 which is linked to a diagnostic agent that is detectable upon imaging.

9. The peptide of claim 8, wherein said diagnostic agent is a contrasting agent, a chromophore, a paramagnetic ion, a radioactive ion or a fluorogenic ion.

10. A diagnostic composition containing the peptide of claim 1, 8 or 9.

11. Use of a peptide according to any one of claims 1 to 4, a polynucleotide of claim 5 or an expression vector of claim 6 for the preparation of a medicament for the treatment of a tumor.

12. Use of a peptide of claim 1, 6 or 7 for the preparation of a diagnostic composition for tumor imaging.

13. Use according to claim 11 or 12, wherein said tumor is a prostate tumor.

## Patentansprüche

1. Peptid zur Verwendung in der Tumortherapie, das zur selektiven Bindung an Tumorzellen fähig ist, wobei das Peptid umfasst
(a) ein Peptid, das die Aminosäuresequenz FRPNRAQDYNTN oder eine Aminosäuresequenz, die sich von der Aminosäuresequenz FRPNRAQDYNTN durch eine oder mehrere konservative Aminosäuresubstitutionen unterscheidet, hat; oder
(b) ein Peptid, das eine Aminosäuresequenz hat, die zumindest 60% Identität zu der Aminosäuresequenz des Peptids von (a) aufweist,
wobei die konservativen Aminosäuresubstitutionen Austausch der aliphatischen oder hydrophoben Aminosäuren Ala, Val, Leu und Ile; Austausch der Hydroxyl-Reste Ser und Thr; Austausch der sauren Reste Asp und Glu; Austausch der Amid-Reste Asn und Gln; Austausch der basischen Reste Lys, Arg und His; Austausch der aromatischen Reste Phe, Tyr und Trp, und Austausch der kleinen Aminosäuren Ala, Ser, Thr, Met und Gly sind.

2. Peptid nach Anspruch 1, welches operativ an einen therapeutischen Wirkstoff gebunden ist, der fähig ist, einen therapeutischen Effekt auf einen Tumor auszuüben.

3. Peptid nach Anspruch 2, wobei der therapeutische Wirkstoff ein antizellulärer Wirkstoff, chemotherapeutischer Wirkstoff, ein Radioisotop oder ein Cytotoxin ist.

4. Peptid nach Anspruch 3, wobei das Cytotoxin ein A-Kettentoxin, ein Ribosominaktivierendes Protein, α-Sarcin, Aspergillin, Restrictotin, Diphterietoxin, Pseudomonas Exotoxin, ein bakterielles Endotoxin oder der Lipid A-Teil eines bakteriellen Toxins ist.

5. Polynukleotid, das das Peptid nach Anspruch 1 kodiert.

6. Expressionsvektor, der das Polynukleotid nach Anspruch 5 enthält.

7. Pharmazeutische Zusammensetzung enthaltend ein Peptid nach einem der Ansprüche 1 bis 4, ein Polynukleotid nach Anspruch 5 oder einen Expressionsvektor nach Anspruch 6.

8. Peptid nach Anspruch 1, das mit einem diagnostischen Wirkstoff, der über Bildverarbeitung detektierbar ist, verbunden ist.

9. Peptid nach Anspruch 8, wobei der diagnostische Wirkstoff ein Kontrastreagens, ein Chromophor, ein paramagnetisches Ion, ein radioaktives Ion oder ein fluorogenes Ion ist.

10. Diagnostische Zusammensetzung enthaltend das Peptid nach Anspruch 1, 8 oder 9.

11. Verwendung eines Peptids nach einem der Ansprüche 1 bis 4, eines Polynukleotids nach Anspruch 5 oder eines Expressionsvektors nach Anspruch 6 zur Herstellung eines Medikaments für die Behandlung eines Tumors.

12. Verwendung eines Peptids nach Anspruch 1, 6 oder 7 zur Herstellung einer diagnostischen Zusammensetzung für die Tumordarstellung.

13. Verwendung nach Anspruch 11 oder 12, wobei der Tumor ein Prostatatumor ist.

## Revendications

1. Peptide destiné à être utilisé dans la thérapie tumorale étant capable de se lier de manière sélective à des cellules tumorales, dans lequel ledit peptide comprend
(a) un peptide possédant la séquence d'acides aminés FRPNRAQDYNTN ou une séquence d'acides aminés qui diffère de la séquence d'acides aminés FRPNRAQDYNTN par une ou plusieurs substitutions d'acides aminés conservatrices ; ou
(b) un peptide possédant une séquence d'acides aminés qui présente au moins 60 % d'identité à la séquence d'acides aminés du peptide de (a), dans lequel lesdites substitutions d'acides aminés conservatrices sont un remplacement des acides aminés aliphatiques ou hydrophobes Ala, Val, Leu et Ile ; un remplacement des résidus hydroxyle Ser et Thr ; un remplacement des résidus acides Asp et Glu ; un remplacement des résidus amide Asn et Gln; un remplacement des résidus basiques Lys, Arg et His; un remplacement des résidus aromatiques Phe, Tyr et Trp, et un remplacement des acides aminés de petite taille Ala, Ser, Thr, Met et Gly.

2. Peptide selon la revendication 1 qui est relié de manière opérationnelle à un agent thérapeutique capable d'exercer un effet thérapeutique sur une tumeur.

3. Peptide selon la revendication 2, dans lequel ledit agent thérapeutique est un agent anti-cellulaire, un agent chimiothérapeutique, un radio-isotope ou une cytotoxine.

4. Peptide selon la revendication 3, dans lequel ladite cytotoxine est une toxine à chaîne A, une protéine d'inactivation de ribosome, l'α-sarcine, l'aspergilline, la restrictotine, la toxine de la diphtérie, l'exotoxine de Pseudomonas, une endotoxine bactérienne ou la fraction de lipide A d'une endotoxine bactérienne.

5. Polynucléotide codant pour le peptide selon la revendication 1.

6. Vecteur d'expression contenant le polynucléotide selon la revendication 5.

7. Composition pharmaceutique contenant un peptide selon l'une quelconque des revendications 1 à 4, un polynucléotide selon la revendication 5 ou un vecteur d'expression selon la revendication 6.

8. Peptide selon la revendication 1 qui est lié à un agent diagnostique qui est détectable par imagerie.

9. Peptide selon la revendication 8, dans lequel ledit agent diagnostic est un produit de contraste, un chromophore, un ion paramagnétique, un ion radioactif ou un ion fluorogénique.

10. Composition diagnostique contenant le peptide selon la revendication 1, 8 ou 9.

11. Utilisation d'un peptide selon l'une quelconque des revendications 1 à 4, d'un polynucléotide selon la revendication 5 ou d'un vecteur d'expression selon la revendication 6 pour la préparation d'un médicament pour le traitement d'une tumeur.

12. Utilisation d'un peptide selon la revendication 1, 6 ou 7 pour la préparation d'une composition diagnostique pour l'imagerie tumorale.

13. Utilisation selon la revendication 11 ou 12, dans laquelle ladite tumeur est une tumeur de la prostate.
